# EUROPÄISCHE PATENTANMELDUNG

(11) **EP 4 317 938 A1**
(43) Veröffentlichungstag der Anmeldung: **07.02.2024**
(21) Anmeldenummer: 22020372.3
(22) Anmeldetag: 03.08.2022
(51) Int. Cl.: G01N 1/22, G01N 15/06, G01N 33/00

(54) **MOBILES SYSTEM UND VERFAHREN ZUR ZEITLICH UND RÄUMLICH HOCH AUFGELÖSTEN CHARAKTERISIERUNG VON PARTIKELFÖRMIGEN LUFTSCHADSTOFFEN**

(71) Anmelder: Leibniz-Institut für Troposphärenforschung e.V., 04318 Leipzig (DE)
(72) Erfinder: KAETHNER, Ralf, 04229 Leipzig (DE)
(74) Vertreter: Völger, Karl Wolfgang

(57) **Zusammenfassung**

Die vorliegende Erfindung bezieht sich auf ein mobiles System (1) zur zeitlich und räumlich hoch aufgelösten Charakterisierung von partikelförmigen Luftschadstoffen, umfassend
- zumindest einen ersten Sensor (101) zur Erfassung von Rußpartikeln,
- zumindest einen zweiten Sensor (103) zur Erfassung der Gesamtpartikelmasse (PM2.5) von Partikeln mit einem Durchmesser kleiner 2,5 µm,
- zumindest einen dritten Sensor (105) zur Erfassung von meteorologischen Parametern,
- zumindest eine Datenerfassung mit einer Schnittstelle zur drahtlosen Datenübertragung an mobile Endgeräte in einem Datennetzwerk,
- zumindest eine mobile Energieversorgung (117) und
- zumindest eine komplette Dateninfrastruktur (115) zur zentralisierten Datenverarbeitung und Datenspeicherung,
wobei das mobile System (1) in einer von einer Person tragbaren Einheit untergebracht ist.

Die vorliegende Erfindung betrifft ferner ein Verfahren zur zeitlich und räumlich hoch aufgelösten Charakterisierung von partikelförmigen Luftschadstoffen.

## Beschreibung

Die vorliegende Erfindung bezieht sich auf ein mobiles System und ein Verfahren zur zeitlich und räumlich hoch aufgelösten Charakterisierung von partikelförmigen Luftschadstoffen.

Das Problem "saubere Luft" betrifft grundsätzlich jeden Menschen, insbesondere jenen, der in urbaner Umgebung lebt. Die Erfassung von Umweltparametern im Allgemeinen sowie von Luftschadstoffkonzentrationen im Speziellen ist ein hoch relevantes Thema, das durch die zunehmende Verstädterung (Urbanisierung) auch noch weiter an Bedeutung gewinnt. Mit der hohen Bevölkerungsdichte verbunden ist eine Zunahme von klimatischen, umweltbedingten, ökonomischen und epidemiologischen Stressfaktoren. Speziell das Thema Luftqualität ist dabei ein wesentlicher und aktueller Faktor.

Aufgrund der hohen Relevanz ist die maximal zulässige Konzentration zahlreicher Luftschadstoffe gesetzlich geregelt. Zu diesen zählen vor allem Ruß ("eBC" = equivalent black carbon) und die Partikelgesamtmassenkonzentrationen des Feinstaubs ("PM" = particulate matter) mit einem aerodynamischen Durchmesser kleiner als 2,5 µm ("PM2.5") bzw. kleiner als 10 µm ("PM10"). Für PM10 gilt dabei die EU-Richtlinie 1999/30/EG, für PM2.5 die Richtlinie 2008/50/EG. Beide Richtlinien geben Obergrenzen für Tagesmittelwerte, die Richtlinie 1999/30/EG außerdem für Jahresmittelwerte. Ruß ist, obwohl keine gesetzlich verbindlichen Grenzwerte existieren, nachgewiesenermaßen toxisch. Die Exposition gegenüber hohen Konzentrationen kann weitreichende negative Auswirkungen auf die Gesundheit haben.

Für die Überwachung und Einhaltung der Grenzwerte sind in der Bundesrepublik Deutschland die jeweiligen Bundesländer verantwortlich, bei Überschreitungen müssen Maßnahmen getroffen werden. Daher betreiben die Bundesländer, bzw. deren zuständige Umweltämter, Immissionsmessnetze. Die Messungen erfolgen an wenigen Standorten mit kostenintensiven, stationären Messstationen.

Die Frage nach der individuellen Relevanz bestehender Messnetze mit grober Auflösung ist nicht abschließend geklärt. Durch fortschreitende Entwicklungen in Schlüsseltechnologien wie Informationstechnologie oder Mikroelektronik wird der Wunsch der Kontrolle individueller Exposition gegenüber Luftschadstoffen zunehmend erfüllbar.

Die Erhebung von Umweltmessdaten gewinnt seit einigen Jahren zunehmend weiter an Bedeutung. Ein immer größer werdender Anteil der Bevölkerung lebt in größeren Städten oder deren unmittelbarem Umfeld. Zusätzlich zu den vorstehend schon genannten klimatischen, umweltbedingten, ökonomischen und epidemiologischen Stressfaktoren erfordert die zunehmend älter werdende Bevölkerung Maßnahmen zur Aufrechterhaltung der Gesundheit. Dazu gehört auch saubere Luft und damit die Definition und Überwachung von Grenzwerten. Der Zusammenhang zwischen hohen Konzentrationen von Luftschadstoffen und gesundheitlichen Problemen wurde in den letzten Jahren wissenschaftlich untersucht und belegt. Ein weiterer Punkt für die zunehmende Bedeutung der Umweltdatenerhebung ist das zunehmende Bewusstsein der Menschen gegenüber den klimatischen Veränderungen. Das Interesse und damit der Bedarf an Messungen ist, zusammen mit den Möglichkeiten, in den letzten Jahren gestiegen.

Das Problem der mobilen und hoch aufgelösten Charakterisierung von Ruß (eBC) und Feinstaub (PM) wurde daher bis vor einigen Jahren gar nicht gelöst. Die wenigen wissenschaftlich verfügbaren Geräte waren groß, schwer und komplex in der Handhabung. Erst mit den Fortschritten in den Schlüsseltechnologien Mikroelektronik und Informationstechnologie wurden einfachere Lösungen machbar.

Seit einiger Zeit existieren verschiedene Lösungen für Teilaspekte. Beispielsweise ist der Markt für kleine Geräte zur Erfassung der Partikelmassenkonzentration (PM) inzwischen praktisch unüberschaubar geworden. Grundsätzlich arbeiten diese Geräte meist alle nach demselben optischen Prinzip der Laserlichtstreuung. Die Entwicklungen sind begrüßenswert, allerdings fehlt es diesen Geräten meist an Datenqualität. Der Grund dafür liegt nicht an der Sensorik an sich, sondern an mikrophysikalischen Prozessen der (Feinstaub-)Partikel und der Notwendigkeit einer Messgasaufbreitung, z.B. um Effekte der relativen Luftfeuchte korrigieren zu können.

Wissenschaftliche Geräte sind dagegen zumeist sehr stark auf den zu erfassenden Parameter und die Prozesse fokussiert. Ihnen fehlt es immer noch oft an einer einfachen Bedienung, einer intuitiven Visualisierung in Echtzeit oder einer kompletten Dateninfrastruktur zur Prozessierung oder Analyse der Messdaten. Aktuell ist den Erfindern kein Gerät zur mobilen Erfassung der Rußkonzentration bekannt, welches die genannten Eigenschaften aufweist.

Wie vorstehend dargelegt, waren bisherige Lösungen auf Teilaspekte beschränkt. Eine einfach zu bedienende, mobile und hoch aufgelöste, qualitätsgesicherte Erfassung der urbanen eBC und PM2.5 Konzentration inkl. einer Echtzeitdarstellung der Messdaten ist damit nicht möglich.

Bisherige Lösungen setzen oft einen versierten Anwender voraus (z.B. Wissenschaftler oder geschultes Personal), sind eher für stationäre Anwendungen vorgesehen (zu schwer, zu groß), liefern keine ausreichende Datenqualität (ultra-low-cost Sensoren, fehlende Messgasaufbereitung), erfassen nicht alle Parameter (nur PM2.5/PM10 oder nur eBC), besitzen keine oder unzureichende Echtzeitvisualisierung oder sind nur für Einzelanwendung vorgesehen (nur Probennahme, keine Webanwendung und Vernetzung).

Damit bieten bisherige Lösungen keine hinreichende Möglichkeit zur Charakterisierung einer individuellen Exposition gegenüber den genannten Luftschadstoffen und sind auch nicht für ein breites Feld von Anwendern geeignet. Zudem fehlt es den bisherigen Lösungen entweder an Datenqualität (aufgrund kostengünstiger und einfacher Komponenten) oder an Anwenderfreundlichkeit gepaart mit wirtschaftlichen Aspekten (aufgrund teurer wissenschaftlicher Geräte mit oft prototypischem Einsatz und der Notwendigkeit aufwendiger Datenanalyse individuell durch Experten).

Es besteht daher Bedarf an einem mobilen Messsystem, mit dem die Nachteile des Standes der Technik überwunden werden können und das als einfach zu bedienendes, skalierbares System eine Grundlage für qualitätsgesicherte Messungen durch Wissenschaftler, Behörden aber auch interessierte Bürger bildet.

Der vorliegenden Erfindung liegt daher die Aufgabe zugrunde, ein neuartiges mobiles System zur zeitlich und räumlich hoch aufgelösten Charakterisierung von partikelförmigen Luftschadstoffen bereitzustellen, das die genannten Nachteile des Standes der Technik beseitigt, die angestrebte Charakterisierung in einem kompakten und mobilen Messgerät kombiniert sowie mittels moderner Informationstechnologie verknüpft. Es ist ferner Aufgabe der vorliegenden Erfindung, ein Verfahren zur zeitlich und räumlich hoch aufgelösten Charakterisierung von partikelförmigen Luftschadstoffen anzugeben.

Diese Aufgabe wird in einem ersten Aspekt der vorliegenden Erfindung durch ein mobiles System (1) zur zeitlich und räumlich hoch aufgelösten Charakterisierung von partikelförmigen Luftschadstoffen gelöst, umfassend
- zumindest einen ersten Sensor (101) zur Erfassung von Rußpartikeln,
- zumindest einen zweiten Sensor (103) zur Erfassung der Gesamtpartikelmasse (PM2.5) von Partikeln mit einem Durchmesser kleiner 2,5 µm,
- zumindest einen dritten Sensor (105) zur Erfassung von meteorologischen Parametern,
- zumindest eine Datenerfassung mit einer Schnittstelle zur drahtlosen Datenübertragung an mobile Endgeräte in einem Datennetzwerk,
- zumindest eine mobile Energieversorgung (117) und
- zumindest eine komplette Dateninfrastruktur 115 (als Datennetzwerk) zur zentralisierten Datenverarbeitung und Datenspeicherung,
wobei das mobile System (1) in einer von einer Person tragbaren Einheit untergebracht ist.

Unter "zeitlich und räumlich hoch aufgelöster Charakterisierung" wird erfindungsgemäß eine Messung pro Sekunde (ein Hertz) verstanden.

"Partikelförmige Luftschadstoffe" sind hier insbesondere die Gesamtpartikelmasse (PM2.5) von Partikeln mit einem Durchmesser kleiner 2,5 µm, sowie die Masse an Rußpartikeln (eBC).

Zu den "meteorologischen Parametern" zählen erfindungsgemäß vor allem Temperatur, Feuchtigkeit, Luftdruck.

Sensoren zur Erfassung von Rußpartikeln und zur Erfassung der Gesamtpartikelmasse PM2.5 sind grundsätzlich bekannt. In der vorliegenden Erfindung werden bevorzugt solche Sensoren MA200 (eBC, Aethlabs, San Francisco) und PMS5003 (PM2.5, Plantower, modifiziert) verwendet.

Mit "Datenerfassung" wird erfindungsgemäß die automatisierte Datenübertragung vom Messgerät über Smartphone bis zum Webserver und der Online-Plattform, die Datenverarbeitung zur Aufbereitung mittels automatisierter Skripte und die Datenspeicherung in einer InfluxDB bezeichnet, während ferner die "Schnittstelle zur drahtlosen Datenübertragung" vorhanden ist, welche sowohl die Verbindung zwischen mobilem Messgerät per Bluetooth sowie die Übertragung zum Webserver über mobiles Internet (GSM) sicherstellt.

Das erfindungsgemäße mobile System (1) zeichnet sich neben seiner neuartigen Zusammenstellung der einzelnen Komponenten insbesondere dadurch aus, dass es in einer von einer Person tragbaren Einheit untergebracht ist.

Bei der Person handelt es insbesondere sich um einen durchschnittlichen Erwachsenen. Die "tragbare Einheit", deren Größe die Maße von 28 cm x 13 cm x 8,5 cm (Höhe x Breite x Tiefe) nicht überschreitet und deren Gewicht nicht mehr als 1,5 kg beträgt, kann bevorzugt in einem Rucksack / Messrucksack untergebracht sein.

Das mobile System (1) der vorliegenden Erfindung erfüllt alle vorstehend genannten Anforderungen, insbesondere eine zeitlich und räumlich hoch aufgelöste Charakterisierung von partikelförmigen Luftschadstoffen in Bezug auf eine individuelle Exposition gegenüber diesen. Nach Kenntnisstand der Erfinder können mit der vorliegenden Erfindung erstmals zeitlich hoch aufgelöste Rußmessungen mit PM2.5-Messungen in einem kompakten, mobilen Messgerät kombiniert und mittels moderner Informationstechnologie verknüpft werden.

Das erfindungsgemäße mobile System (1) ist in der Lage, Daten in wissenschaftlicher Qualität für PM2.5 und Ruß ohne besondere Vorkenntnisse des Anwenders zu liefern. Basis für den Messrucksack sind neuartige Sensoren zur Erfassung von Umweltparametern. Durch fortschreitende Entwicklungen auf dem Gebiet der Mikroelektronik und Informationstechnologie wurden in den letzten Jahren zahlreiche neue Sensoren auf den Markt gebracht, deren Nachweisgrenze inzwischen derart verbessert wurde, dass damit nun grundsätzlich auch die Überwachung bestimmter Luftqualitätsparameter im urbanen Raum möglich geworden ist. Für einen Einsatz der Sensoren in Messgeräten sind jedoch in der Regel noch weitere, oft aufwändige Maßnahmen wie eine Messgaskonditionierung und eine Nullpunktkorrektur notwendig, d.h. aus Sensoren müssen langzeitstabile, qualitätsgeprüfte Messgeräte entwickelt werden. Auf Basis langjähriger Erfahrungen und mehrerer Entwicklungsstufen haben die Erfinder derartige qualitätssichernde Maßnahmen in das System implementiert.

Ein weiterer wichtiger Aspekt ist die Automatisierung des Systems. Als Werkzeug zur Anwendung sowohl durch Wissenschaftler, aber auch durch Behörden und interessierte Laien konzipiert, dienen eine Mikrokontroller-Steuerung, eine mobile App und die webbasierte Dateninfrastruktur (115) der fast vollständigen Automatisierung. Der Status des Messsystems kann fortlaufend automatisch überwacht und übermittelt werden. Der Anwender muss sich praktisch nicht um technische Aspekte kümmern, sondern kann sich voll auf die Messungen konzentrieren. Auch die Datenspeicherung und Auswertung wurden automatisiert. Die Geräte sind über die Dateninfrastruktur (115) sowie die webbasierte Online-Plattform vernetzt. Diese dient zur erweiterten Darstellung der Messungen, deren Nachbearbeitung, Kommentierung und Einordnung in übergeordneten Kontext (z.B. Großwetterlage) sowie zum Austausch der messenden Personen untereinander in Form eines Forums. Die hinter der vorliegenden Erfindung stehende Technologie ist in der Größe und Anzahl der Teilnehmer skalierbar und leistet erprobtermaßen einen Beitrag zum Wissenstransfer.

Die vorliegende Erfindung eröffnet Anwendungen für wissenschaftliche Institute, die mit dem sog. "Citizen Science" Ansatz arbeiten. Darüber hinaus kann die vorliegende Erfindung als Qualitätsstandard oder wissenschaftliche Referenzanweisung von Startups weltweit eingesetzt werden, die die Genauigkeit ihrer PM2.5- und eBC-Messungen überprüfen und validieren möchten. Das erfindungsgemäße mobile System (1) kann für Forschung und Industrie eingesetzt werden. Das erfindungsgemäße mobile System (1) kann auch als Referenzwerkzeug für andere ähnliche Technologien verwendet oder als wissenschaftliches Luftqualitätsmesssystem für wissenschaftliche Projekte eingesetzt werden.

Obwohl das erfindungsgemäße mobile System (1) nicht als kostengünstig erachtet werden kann, wird mit der vorliegenden Erfindung eine wirtschaftlich sinnvolle Lösung angeboten, welche eine hohe Datenqualität mit einer großen Anwenderfreundlichkeit vereint.

In einer Weiterbildung des erfindungsgemäßen mobilen Systems (1) umfasst dies ferner
- eine Probenflussregelung,
- ein Einlasssystem (119) mit einer Messgasaufbereitung,
- eine Einrichtung zur Nullmessung,
- einen GPS-Empfänger (121).

Mit dieser Weiterbildung kann das erfindungsgemäße mobile System (1) als vollautomatischer Messrucksack (in Form einer tragbaren Einheit) ausgestaltet werden, der mit der Probenflussregelung, der Messgasaufbereitung, der Einrichtung zur Nullmessung und einem Datenlogger, einer App zur Echtzeitvisualisierung sowie einer Anbindung zu einem Webserver mit automatisierter Datenverarbeitung und Datenspeicherung ausgestattet ist.

Eine andere Ausführungsform des erfindungsgemäßen mobilen Systems (1) sieht vor, dass die Dateninfrastruktur (115) aufweist
- zumindest eine redundante Sicherung von Rohdaten auf mobilen Messeinheiten, mobilen Endgeräten sowie auf einem Datenserver,
- zumindest eine drahtlose, automatisierte Datenübertragung zu einem Datenserver,
- zumindest eine automatisierte Prozessierung der Rohdaten, sowie die Speicherung der prozessierten Daten in einer Datenbank.

Der anbindbare Webserver bietet neben der Datenbank eine umfangreiche interaktive Messdatenplattform (mit Webseite) zur erweiterten Bearbeitung und Visualisierung der eigenen Messungen. Die Serverinfrastruktur wiederum dient zur Bündelung der Messungen und als Eingabe für wissenschaftliche Modellierungen.

Das erfindungsgemäße mobile System (1) kann in einer Weiterbildung vorteilhafterweise eine computergestützte Auswerteelektronik mit Anzeigemöglichkeit auf mobilen Endgeräten umfassen.

In dieser Weiterbildung weist die computergestützte Auswerteelektronik bevorzugt auf
- zumindest eine optischen Rückmeldung über den funktionalen Zustand des mobilen Systems (1),
- zumindest eine Anzeige für Statusmeldungen,
- zumindest eine numerische Anzeige aller Messwerte in Echtzeit,
- zumindest eine Darstelleinheit für Zeitreihen von Messwerten in einem bestimmbaren Zeitintervall,
- zumindest einer Kommentar- und Notizbuchfunktion.

Diese Weiterbildung erlaubt die unmittelbare Beurteilung der Messungen.

Es hat sich für die flexible Bearbeitung und Auswertung der Daten als vorteilhaft herausgestellt, wenn das erfindungsgemäße mobile System (1) ferner eine webbasierte Datenplattform zur Vernetzung und Analyse der gewonnenen Daten umfasst.

In dieser Ausführungsform kann die die webbasierte Datenplattform insbesondere aufweisen
- zumindest eine Darstellung der Messreihen in Form von Zeitreihen,
- zumindest eine Darstellung der Messreihen auf einer Karte,
- zumindest eine Einrichtung zur Daten-Bereinigung, Daten-Kommentierung und Daten-Auswertung,
- zumindest eine Einrichtung zur Einbettung der aufbereiteten Modellergebnisse,
- zumindest eine Schnittstelle zur Verlinkung weiterer externer Datenquellen,
- zumindest eine Einrichtung zur Erstellung eines Nutzer-Forums.

Für die Aussagekraft der durchgeführten Messungen hat es sich als vorteilhaft erwiesen, wenn die mit dem zumindest einen dritten Sensor (105) erfassten meteorologischen Parameter ausgewählt sind aus Lufttemperatur, relativer Luftfeuchte und/oder Luftdruck. Diese können in die Datenverarbeitung einbezogen werden.

Eine weitere Ausbildung des erfindungsgemäßen mobilen Systems (1) sieht vor, dass die mobile Energieversorgung (117) eine Systemlaufzeit von mindestens 6 Stunden sicherstellt. Damit wird gewährleistet, dass eine ausreichende Messdauer für aussagekräftige Messungen erreicht wird, ohne das mobile System (1) abschalten oder laden zu müssen.

Wenn in der Beschreibung des erfindungsgemäßen mobilen Systems (1) Verfahrensmerkmale genannt werden, so beziehen sich diese insbesondere auf das erfindungsgemäße Verfahren, wie es nachstehend beschrieben wird. Ebenso beziehen sich gegenständliche Merkmale, die in der Beschreibung des erfindungsgemäßen Verfahrens angeführt werden, auf das erfindungsgemäße mobile System (1).

Die vorstehend genannte Aufgabe wird in einem zweiten Aspekt der vorliegenden Erfindung durch ein Verfahren zur zeitlich und räumlich hoch aufgelösten Charakterisierung von partikelförmigen Luftschadstoffen gelöst, umfassend die Schritte
a) Bereitstellen zumindest eines mobilen Systems (1) wie vorstehend beschrieben wurde,
b) Herstellen der Verbindung mit einem mobilen Endgerät nach Einschalten des mobilen Systems (1),
c) Durchführen des Startvorgangs und der initialen Nullmessung des mobilen Systems (1),
d) automatisiertes Durchführen der mobilen Messungen mit dem mobilen System (1),
e) Ausschalten des mobilen Systems (1) nach Beendigung der Messung,
f) Kontrollieren der automatischen Datenübertragung und ggf. Kopieren der Daten auf einen Server,
g) Nachbereiten der mobilen Messungen, beinhaltend:
   g1) Sichten der Daten auf der online-Plattform,
   g2) Bereinigen der Daten,
   g3) statistische Analyse der Ergebnisse unter Verwendung von Notizen,
   g4) Bewerten der Messergebnisse unter Berücksichtigung der Modellergebnisse.

Das erfindungsgemäße Verfahren weist grundsätzlich die gleichen Vorteile auf wie das erfindungsgemäße mobile System (1), mit dem es ausgeführt wird. Es bietet insbesondere eine zeitlich und räumlich hoch aufgelöste Charakterisierung von partikelförmigen Luftschadstoffen in Bezug auf eine individuelle Exposition gegenüber diesen. Mit dem erfindungsgemäßen System wird der Nutzer in die Lage versetzt, Daten in wissenschaftlicher Qualität für PM2.5 und eBC ohne besondere Vorkenntnisse des Anwenders zu liefern.

Es hat sich als vorteilhaft herausgestellt, wenn in Schritt d) ferner eine Echtzeit-Kontrolle vorgenommen und eine individuelle Notizfunktion bereitgestellt wird.

Darüber hinaus kann in einer Weiterbildung des erfindungsgemäßen Verfahrens das Bereinigen der Daten in Schritt g2) das Löschen von Ausreißern oder Fehlmessungen unter Verwendung der individuellen Notizen umfassen.

Eine besondere Ausführungsform sieht vor, dass in dem erfindungsgemäßen Verfahren zwei oder mehr der erfindungsgemäßen mobilen Systeme (1) über den Datenserver miteinander vernetzt werden und jedes der mobilen Systeme (1) für dessen Datensätze eine feste Identifikationsnummer aufweist, so dass beim Zusammenführen der Daten der zwei oder mehr mobilen Systeme (1) in einer Datenbank eine eindeutige Zuordnung der Messdaten erfolgt.

Mit dieser Ausführungsform ist es möglich, über einen gewissen Bereich für einen gewissen Zeitraum ein Netz von Messdaten zu erheben und miteinander zu verknüpfen. So können beispielsweise örtliche und zeitliche Unterschiede und/oder Entwicklungen von Luftschadstoffbelastungen ermittelt werden.

Schließlich kann das erfindungsgemäße Verfahren vorteilhafterweise ferner die Berechnung eines multi-skaligen und genesteten Modellsystems zur hochaufgelösten urbanen Luftqualitätsvorhersage aufweisen, das zumindest eine 48 h Prognose relevanter partikelförmiger und gasförmiger Luftschadstoffe auf der Basis eines Luftqualitätsindex mit meteorologischen Parametern bezogen auf ein Untersuchungsgebiet umfasst, wobei eine räumliche Auflösung von bis zu 500 m im prognostischen Chemie-Transport-Modell und von bis zu 5 m im dynamischen Downscaling-Verfahren erreicht wird.

Unter einem "multi-skaligen und genesteten Modellsystem" wird im Sinne der vorliegenden Erfindung ein mehrstufiges Modellsystem verstanden, bei dem ausgehend von Simulationen in einem großen Modellgebiet mit grober Auflösung in mehreren Schritten bis zu einem kleinen Modellgebiet mit hoher Auflösung gerechnet wird. Dabei werden die Modellergebnisse des vorherigen Modelllaufes als Eingabe-/Randwerte für die nächste Stufe verwendet.

Mit einem "prognostischen Chemie-Transport-Modell" wird erfindungsgemäß ein Vorhersagemodell für Partikel- und gasförmige Luftschadstoffe und deren Wechselwirkungen bezeichnet.

Ein "dynamisches Downscaling-Verfahren" bedeutet für die vorliegende Erfindung, dass die räumliche Auflösung nicht statisch festgeschrieben ist, sondern dynamisch im Modell bestimmt wird.

Die relevanten partikelförmigen und gasförmigen Luftschadstoffe sind hier speziell PM2.5 und eBC, bzw. Stickoxide NOx und Ozon O₃. Bei den meteorologischen Parametern handelt es sich vor allem um Wind, Temperatur, Feuchte und Luftdruck.

Weitere Ziele, Merkmale, Vorteile und Anwendungsmöglichkeiten ergeben sich aus der nachfolgenden Beschreibung von die Erfindung nicht einschränkenden Ausführungsbeispielen anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigt:

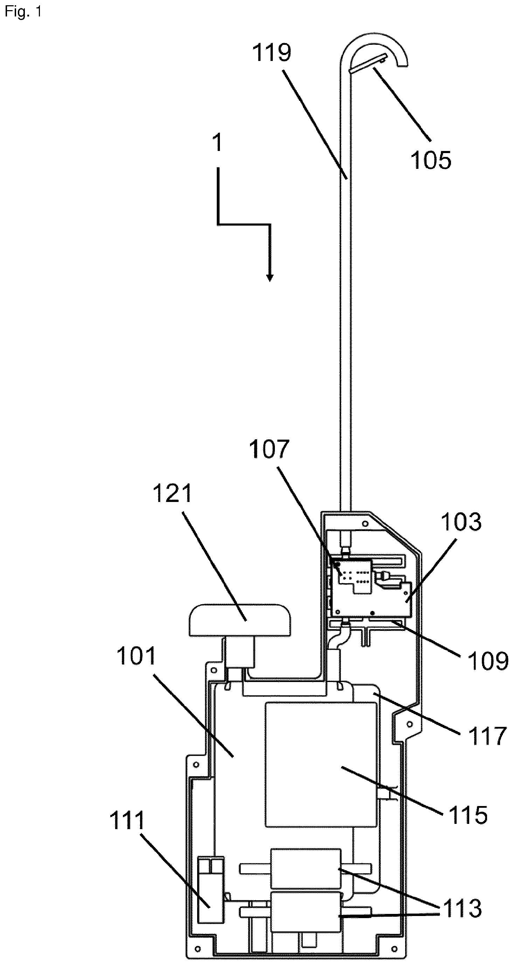

- Fig. 1: eine schematische Darstellung des erfindungsgemäßen mobilen Systems 1 in der Ausführung eines Messungsrucksacks.

In Figur 1 wird schematisch der Messrucksack des erfindungsgemäßen mobilen Sensorsystems visualisiert. Das mobile System 1 zeichnet sich durch die kompakte Anordnung fast aller Komponenten auf engem Raum aus. Lediglich der dritte Sensor 105 zur Erfassung von meteorologischen Parametern befindet sich aus messtechnischen Gründen außerhalb und ist vorzugsweise an dem langgezogenen Einlass 119 angeordnet. Dieser Einlass 119 ragt bei der Verwendung des als Messungsrucksack ausgeführten mobilen Systems 1 nach oben heraus.

Am Ende des Einlasses 119 ist zunächst ein Inline Feuchtesensor 107 angeordnet, mit welchem die Feuchte der zu messenden Luftproben bestimmt wird. Ferner ist dort eine Heizung 109 zur gleichmäßigen Temperierung zu messenden Luftproben vorgesehen.

An dieser Stelle ist der zweite Sensor 103 zur Erfassung der Gesamtpartikelmasse (PM2.5) angeordnet, während der erste Sensor 101 zur Erfassung von Rußpartikeln sich um Hauptteil des mobilen Systems 1 befindet.

Der Hauptteil des mobilen Systems 1 wird unter anderem durch die Dateninfrastruktur (115) (mit Hauptplatine) gebildet, mit welcher die mobile Energieversorgung 117 verbunden ist. Die mobile Energieversorgung 117 besteht bevorzugt aus austauschbaren Akkus, so dass das mobile System nach deren Austausch schnell wieder eingesetzt werden kann. Mit der Dateninfrastruktur 115 ist auch der GPS-Empfänger 121 verbunden.

In der dargestellten Ausführungsform sind ferner eine Pumpe 111 und ein oder mehrere Filter 113 vorgesehen.

Besonders in Städten existiert eine starke kleinräumige Variabilität von Luftschadstoffkonzentrationen. Bedingt wird dies durch eine insgesamt hohe Bevölkerungsdichte und daraus resultierend einer Vielzahl verschiedener anthropogener Emittenten (wie Verkehr, Haushalt oder Gewerbe), aber auch durch die besonders in Städten vorherrschenden komplexen Bebauungsstrukturen (wie Straßenschluchten abgewechselt durch städtische Parkanlagen, etc.). Diese kleinräumige Variabilität der Konzentrationen kurzlebiger Luftschadstoffe kann durch die Immissionsmessstationen der Landesämter nicht detailliert aufgelöst werden. In Folge dessen wird mit den existierenden Messnetzen die Frage nach der individuellen Exposition und den damit verbundenen Auswirkungen auf die Gesundheit nicht abschließend geklärt.

Daher kommen als Ergänzung zu den Immissionsmessnetzen der Landesämter zunehmend auch hochauflösende numerische Ausbreitungsmodelle zum Einsatz, die teilweise die Möglichkeit der Vorhersage der räumlichen und zeitlichen Verteilung der Luftschadstoffe auf Straßenschluchtskala (weniger als 100 m) bieten. Zur Validierung und Weiterentwicklung dieser Modelle sind allerdings räumlich hoch aufgelöste Messungen erforderlich. Die bestehenden Messnetze sind dafür unzureichend.

Damit gewinnt die Notwendigkeit einer umfassenden Erhebung von Umweltdaten aufgrund der Zunahme von klimatischen, umweltbedingten, ökonomischen und epidemiologischen Stressfaktoren auch weiterhin noch an Bedeutung und speziell das Thema Luftqualität ist dabei ein wesentlicher und aktueller Faktor. Potentielle Geräte für die Erhebung der Daten müssen dabei einfach bedienbar sein und möglichst kostengünstig zuverlässige Daten liefern. Grundsätzlich sind zur Erhöhung der räumlichen Dichte von Immissionsmessungen, je nach Einsatzzweck, stationäre und mobile Geräte geeignet. In unserer modernen, mobilen Welt sind für den Nicht-Experten jedoch besonders kleine, mobile Geräte interessant, welche in Echtzeit Daten erfassen und für den Nutzer zugänglich machen.

Insgesamt ist die Problematik der Erfassung von Luftqualitätsparametern ein komplexes Thema. Insbesondere Messungen von Schwarzkohlenstoff (Ruß) mit hoher zeitlicher Auflösung ist sehr anspruchsvoll. Weiterhin bilden einzelne Sensoren auch noch kein Messgerät. Wissenschaftliche Geräte sind zumeist kostenintensiv, groß und schwer, sowie kompliziert in der Bedienung, während kostengünstige Geräte keine ausreichende Datenqualität aufweisen.

Nachstehend wird vor diesem Hintergrund die vorliegende Erfindung anhand einer bevorzugten Ausführungsform erläutert, auf welche die Erfindung aber nicht beschränkt ist.

Die vorliegende Erfindung betrifft ein innovatives, mobiles System 1 - als eine Art mobiles Messsystem - zur Charakterisierung der Luftqualität im urbanen Raum. Erfasst werden dabei die gesundheitlich hoch relevante Massenkonzentration von Rußpartikeln eBC sowie die gesetzlich regulierten partikelförmigen Luftschadstoffe PM2.5 und PM10. Wie Ebenfalls gemessen werden die grundlegenden meteorologischen Parameter Lufttemperatur, relative Luftfeuchte und Luftdruck.

Das mobile Messsystem 1 stellt die aktuellste Entwicklungsstufe mobiler Partikelmessgeräte der Erfinder am Leibniz Institut für Troposphärenforschung (TROPOS) als Anmelder dar. Das TROPOS beschäftigt sich als außeruniversitäre Forschungseinrichtung der Leibniz-Gesellschaft seit seiner Gründung im Jahre 1992 mit Untersuchungen in der belasteten Atmosphäre und ist international anerkannter Spezialist auf dem Gebiet von Aerosolen, Wolken und deren Wechselwirkungen. Ein wesentlicher Anspruch am TROPOS ist die Erhebung qualitätsgesicherter Daten.

Die Innovation des neuartigen mobilen Messsystems 1 ist die Kombination aus miniaturisierter Umwelt-Sensorik und langjähriger Expertise des TROPOS auf dem Gebiet der Aerosolmesstechnik, eingebettet in eine moderne Dateninfrastruktur 115 mit einer webbasierten Plattform zur Vernetzung und Datenanalyse.

Die Kombination verschiedener Sensoren und Geräte in einem Komplettsystem in Form eines leichten Messrucksacks mit einer ausdauernden Akkulaufzeit bildet zusammen mit der mobilen App, sowie der kompletten Dateninfrastruktur 115 und der online Plattform ein innovatives System zur qualitätsgesicherten Überwachung der Ruß- und Partikelmassenkonzentration, welches von Wissenschaftlern, Behörden und interessierten Bürgern genutzt werden kann. Das gesamte System ist in der Größe und Anzahl der Teilnehmer skalierbar und leistet erprobtermaßen einen Beitrag zum Wissenstransfer.

Mit dem mobilen System 1 der vorliegenden Erfindung, eingebettet in einen Messrucksack, bietet sich, nach aktuellem Kenntnisstand, zum ersten Mal die Möglichkeit, in einem kompakten Gehäuse mit hoher Qualität sowohl die gesundheitlich hoch relevante Konzentration von Schwarzkohlenstoff (Ruß) und gleichzeitig ein Äquivalent der Gesamtpartikelmasse (PM10/PM2.5) mit sehr hoher zeitlicher Auflösung von 1 Sekunde (im Gegensatz zu Stundenmittelwerten der Immissionsmessstationen) in Echtzeit zu erfassen und dem Nutzer zugänglich zu machen.

Mit einer implementierten wartungsfreien Messgasaufbreitung, d.h. einer Heizung zur Trocknung, sowie der Möglichkeit einer Nullpunktkorrektur, d.h. Filter-/Nullpunktmessung, wird dabei auch grundlegenden wissenschaftlichen Standards Rechnung getragen und eine hohe Datenqualität sichergestellt. Das erfindungsgemäße mobile System 1 ermöglicht durch die hohe Mobilität sowohl die Identifizierung lokaler Hot-Spots, als auch die Bestimmung einer individuellen Exposition. Durch die Anbindung an eine mobile App ist die Möglichkeit der Vernetzung und die Anbindung an eine zentrale Datenbank gewährleistet. Außerdem ermöglicht die mobile App die Visualisierung der Messdaten in Echtzeit.

Durch die einfache Bedienbarkeit und Vielseitigkeit auf der einen, sowie die gesicherte Datenqualität auf der anderen Seite bietet das erfindungsgemäße mobile System 1 ein neuartiges und ideales Messsystem zur Anwendung von interessierten Laien, aber auch Behörden und Wissenschaftlern. Mit dem kompakten Gehäuse bietet das System zum ersten Mal die Möglichkeit, derartige Messungen problemlos in den alltäglichen Tagesablauf integrieren zu können. Das System kann damit einen Beitrag zur kleinräumigen Charakterisierung der Immissionsbelastung leisten.

### Bezugszeichen

- 1: mobiles System
- 101: ersten Sensor zur Erfassung von Rußpartikeln
- 103: zweiten Sensor zur Erfassung der Gesamtpartikelmasse (PM2.5)
- 105: dritten Sensor zur Erfassung von meteorologischen Parametern
- 107: Inline Feuchtesensor
- 109: Heizung
- 111: Pumpe
- 113: Filter
- 115: Dateninfrastruktur
- 117: Energieversorgung
- 119: Einlass
- 121: GPS-Empfänger

## Patentansprüche

1. Mobiles System (1) zur zeitlich und räumlich hoch aufgelösten Charakterisierung von partikelförmigen Luftschadstoffen, umfassend
- zumindest einen ersten Sensor (101) zur Erfassung von Rußpartikeln,
- zumindest einen zweiten Sensor (103) zur Erfassung der Gesamtpartikelmasse (PM2.5) von Partikeln mit einem Durchmesser kleiner 2,5 µm,
- zumindest einen dritten Sensor (105) zur Erfassung von meteorologischen Parametern,
- zumindest eine Datenerfassung mit einer Schnittstelle zur drahtlosen Datenübertragung an mobile Endgeräte in einem Datennetzwerk,
- zumindest eine mobile Energieversorgung (117) und
- zumindest eine komplette Dateninfrastruktur (115) zur zentralisierten Datenverarbeitung und Datenspeicherung,
wobei das mobile System (1) in einer von einer Person tragbaren Einheit untergebracht ist.

2. Mobiles System (1) nach Anspruch 1, ferner umfassend
- eine Probenflussregelung,
- ein Einlasssystem mit einer Messgasaufbereitung,
- eine Einrichtung zur Nullmessung,
- einen GPS-Empfänger (121).

3. Mobiles System (1) nach Anspruch 1 oder 2, wobei die Dateninfrastruktur (115) aufweist
- zumindest eine redundante Sicherung von Rohdaten auf mobilen Messeinheiten, mobilen Endgeräten sowie auf einem Datenserver,
- zumindest eine drahtlose, automatisierte Datenübertragung zu einem Datenserver,
- zumindest eine automatisierte Prozessierung der Rohdaten, sowie die Speicherung der prozessierten Daten in einer Datenbank.

4. Mobiles System (1) nach einem der Ansprüche 1 bis 3, ferner umfassend eine computergestützte Auswerteelektronik mit Anzeigemöglichkeit auf mobilen Endgeräten.

5. Mobiles System (1) nach Anspruch 4, wobei die computergestützte Auswerteelektronik aufweist
- zumindest eine optische Rückmeldung über den funktionalen Zustand des mobilen Systems (1),
- zumindest eine Anzeige für Statusmeldungen,
- zumindest eine numerische Anzeige aller Messwerte in Echtzeit,
- zumindest eine Darstelleinheit für Zeitreihen von Messwerten in einem bestimmbaren Zeitintervall,
- zumindest einer Kommentar- und Notizbuchfunktion.

6. Mobiles System (1) nach einem der Ansprüche 1 bis 5, ferner umfassend eine webbasierte Datenplattform zur Vernetzung und Analyse der gewonnenen Daten.

7. Mobiles System (1) nach Anspruch 6, wobei die webbasierte Datenplattform aufweist
- zumindest eine Darstellung der Messreihen in Form von Zeitreihen,
- zumindest eine Darstellung der Messreihen auf einer Karte,
- zumindest eine Einrichtung zur Daten-Bereinigung, Daten-Kommentierung und Daten-Auswertung,
- zumindest eine Einrichtung zur Einbettung der aufbereiteten Modellergebnisse,
- zumindest eine Schnittstelle zur Verlinkung weiterer externer Datenquellen,
- zumindest eine Einrichtung zur Erstellung eines Nutzer-Forums.

8. Mobiles System (1) nach einem der Ansprüche 1 bis 7, wobei die mit dem zumindest einen dritten Sensor (105) erfassten meteorologischen Parameter ausgewählt sind aus Lufttemperatur, relativer Luftfeuchte und/oder Luftdruck.

9. Mobiles System (1) nach einem der Ansprüche 1 bis 8, wobei die mobile Energieversorgung (117) eine Systemlaufzeit von mindestens 6 Stunden sicherstellt.

10. Verfahren zur zeitlich und räumlich hoch aufgelösten Charakterisierung von partikelförmigen Luftschadstoffen, umfassend die Schritte
a) Bereitstellen zumindest eines mobilen Systems (1) nach einem der Ansprüche 1 bis 9,
b) Herstellen der Verbindung mit einem mobilen Endgerät nach Einschalten des mobilen Systems (1),
c) automatisiertes Durchführen des Startvorgangs und der initialen Nullmessung des mobilen Systems (1),
d) Durchführen der mobilen Messungen mit dem mobilen System (1),
e) Ausschalten des mobilen Systems (1) nach Beendigung der Messung,
f) Kontrollieren der automatischen Datenübertragung und ggf. Kopieren der Daten auf einen Server,
g) Nachbereiten der mobilen Messungen, beinhaltend:
g1) Sichten der Daten auf der online-Plattform,
g2) Bereinigen der Daten,
g3) statistische Analyse der Ergebnisse unter Verwendung von Notizen,
g4) Bewerten der Messergebnisse unter Berücksichtigung der Modellergebnisse.

11. Verfahren nach Anspruch 10, wobei in Schritt d) ferner eine Echtzeit-Kontrolle vorgenommen und eine individuelle Notizfunktion bereitgestellt wird.

12. Verfahren nach Anspruch 10 oder 11, wobei das Bereinigen der Daten in Schritt g2) das Löschen von Ausreißern oder Fehlmessungen unter Verwendung der individuellen Notizen umfasst.

13. Verfahren nach einem der Ansprüche 10 bis 12, wobei zwei oder mehr mobile Systeme (1) nach einem der Ansprüche 1 bis 9 miteinander vernetzt werden und jedes der mobilen Systeme (1) für dessen Datensätze eine feste Identifikationsnummer aufweist, so dass beim Zusammenführen der Daten der zwei oder mehr mobilen Systeme (1) in einer Datenbank eine eindeutige Zuordnung der Messdaten erfolgt.

14. Verfahren nach einem der Ansprüche 10 bis 13, das ferner die Berechnung eines multi-skaligen und genesteten Modellsystems zur hochaufgelösten urbanen Luftqualitätsvorhersage aufweist, das zumindest eine 48 h Prognose relevanter partikelförmiger und gasförmiger Luftschadstoffe auf der Basis eines Luftqualitätsindex mit meteorologischen Parametern bezogen auf ein Untersuchungsgebiet umfasst, wobei eine räumliche Auflösung von bis zu 500 m im prognostischen Chemie-Transport-Modell und von bis zu 5 m im dynamischen Downscaling-Verfahren erreicht wird.
